# EUROPEAN PATENT SPECIFICATION

(11) **EP 0 434 699 B1**
(45) Date of publication and mention of the grant of the patent: **01.12.1993**
(21) Application number: 89908969.2
(22) Date of filing: 14.07.1989
(51) Int. Cl.: A61F 5/451, F16L 37/24

(54) **VALVE AND COUPLING DEVICE FOR USE BETWEEN A URINE CATHETER AND A URINE BAG**
VENTIL UND KUPPLUNGSANORDNUNG ZUR VERWENDUNG ZWISCHEN EINEM HARNKATHETER UND EINEM HARNSACK
DISPOSITIF A VALVE ET A ACCOUPLEMENT DESTINE A ETRE UTILISE ENTRE UN CATHETER URETRO-VESICAL ET UNE POCHE POUR URINE

(43) Date of publication of application: 03.07.1991
(73) Proprietor: TEKNOPLAST A/S, DK-5100 Odense C (DK)
(72) Inventor: HALLERBÄCK, Stig, Lennart, S-424 74 Västra Frölunda (SE)
(74) Representative: Roerboel, Leif
(86) International application number: DK8900176
(87) International publication number: WO9101120

(56) References cited:
- CH-C- 550 354
- GB-A- 2 061 466
- Derwent's abstract No. D7 750 B/17, SU 611 069, publ. week 7917 (KOLPINO BR METALLUR)

## Description

### TECHNICAL FIELD

The present invention relates to a valve and coupling device for connecting a urine catheter with a urine bag and comprising
a) a first coupling part embracing and being movable relative to a valve member with at least one valve aperture capable of being
   moved into an open or closed position respectively by movement of the valve member relative to the first coupling member partly embracing it in a liquid-tight manner, and
b) a second coupling member.

### BACKGROUND ART

Catheters to be introduced in the urethra with a view to emptying the urine bladder are generally connected to a bag with a flexible tube in order to collect the urine. Usually, the connecting operation is relatively simple, but suffers at the same time from great inconveniences, because there is no valving function suited for the purpose, and because the requirements with respect to hygiene and sanitation are ignored to a greater or lesser degree. Thus, the most common way of coupling the bag to the catheter is merely based on the use of a plastic mouthpiece on the bag tube, adapted to be inserted in the free open end of the catheter tube. There is no stop valve, for which reason the operator during the coupling operation has to compress the catheter tube with one hand, so that the flow of urine is stopped during the time interval required, firstly to remove the urine bag having been filled with urine, secondly to replace it with a new and empty urine bag - or alternatively place a clip or clamp on the catheter tube, with the consequent risk of forgetting to remove the clip or clamp after the changing of the bag. Thus the coupling operation proceeds in a number of steps, that may cause problems as seen from a sanitary point of view. Leaking urine is in it self unpleasant and a source of infections. In spite of special training, the hospital personnel have difficulties in changing urine bags, and patients changing urine bags themselves encounter even greater difficulties.

The ideal solution would be a simple and low-cost coupling device comprising a valve making it possible for the operator to use both hands for changing the urine bags without any risk of spilling urine.

Such a coupling device with a valve and of the kind referred to initially is known from GB patent specification No. 2,061,466. Here, however, the coupling function and the valving function are separate. The reason for this is that this coupling device is adapted to be placed in the bottom of a urine bag and to make it possible to empty the latter into a container as an alternative to changing the bag. If this known coupling device were to be placed between the catheter and the urine bag, the separation between the two functions mentioned would open a possibility for faulty operation.

Other systems exist on the market, but they are generally large and clumsy, and their function sequence is uncertain and completely faulty. For this reason, leakage of urine occurs frequently, and since the cost of production is also high, these systems have only been used to a very small extent.

### DISCLOSURE OF THE INVENTION

On this background, it is the object of the present invention to provide a device of the kind referred to initially, in which the coupling and valving functions are integrated, which is small and can be manufactured at a low cost, and which additionally has a function sequence giving practically 100% protection against leakage.

According to the present invention, the above object is achieved with a valve and coupling device of the kind referred to initially, according to the present invention being characterized in that the second coupling member is provided with driving and locking members for releasable coupling engagement with the first coupling member, said driving and locking members being constituted by the same at least one pin adapted to cooperate with driving slots in the valve member and bayonet slots in the first coupling member in such a manner, that the valve apertures are in a closed position, when the bayonet coupling is in a position for separation, and in an open position, when the bayonet coupling is in a locked-together position.

With such a device, it is ensured in a simple and low-cost manner, that the locking connection between the two coupling members is always present when the valve is open. For this reason, practically no spilling of urine occurs, and the operator is free to use both hands to the changing of bags per se, without having to use extra implements, such as clips or clamps.

### BRIEF DESCRIPTION OF THE DRAWINGS

In the following detailed specification, the present invention will be explained in more detail with reference to an exemplary embodiment shown in the drawings, in which
Figure 1 is a side view, partly in longitudinal section, showing an exemplary embodiment of a coupling and valve device according to the present invention,
Figure 2 illustrates the function of the valve in the open position, and
Figure 3 illustrates the function of the valve in the closed position.

### DESCRIPTION OF THE PREFERRED EMBODIMENT

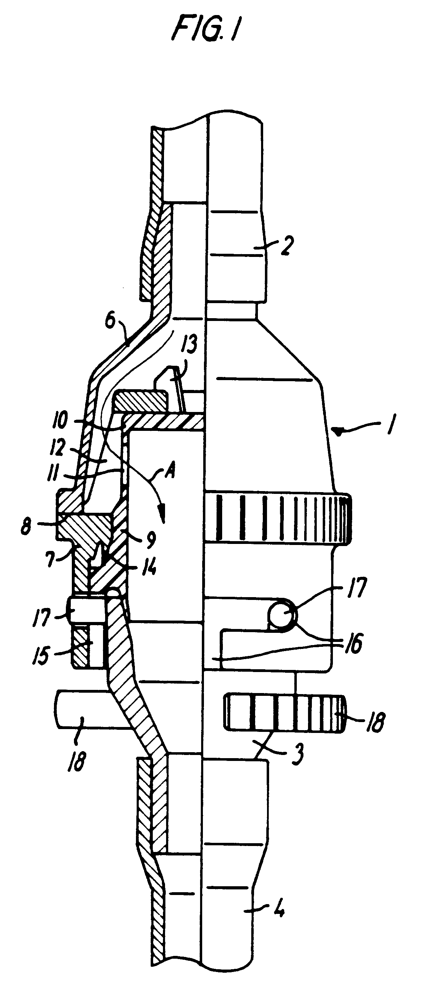

The device shown in Figure 1 comprises a valve and coupling housing 1 connected to the flexible catheter tube 2 and a male coupling member 3 fitted to the flexible tube 4 from the urine bag. The coupling housing 1 is composed of two units, viz. a hood 6 and a valve and female coupling member 7, these two members being joined by welding in a joint 8. The valve and female coupling member 7 comprises an internal valve sleeve 9 rotatable about its longitudinal axis coinciding with the longitudinal axis of the device and between two longitudinal radial bearing and closure members 12, projecting from the female coupling member 7. By turning e.g. through 90°, valve apertures 11 at one end 10 of the valve sleeve 9 are brought into or away from a position facing the closure members 12, so that the valve is closed or opened respectively for the flow (the arrow A) of urine. At the one end 10 mentioned, the valve sleeve 9 is held axially in position by means of a resilient catch 13. At the other end, the valve sleeve 9 is adapted to cooperate with a sealing member 14 in the valve and female coupling member 7, and comprises at least one axially extending actuating slot 15.

In the female coupling member 7 there are bayonet slots 16 in a number equal to the number of actuating slots 15. A similar number of pins 17 are situated on the male coupling member 3.

When the coupling housing 1 and the male coupling member 3 are separate from each other, the valve sleeve 9 is in the closed position, and the actuating slots 15 and the bayonet slots 16 are aligned with each other in pairs. When the two members are to be coupled to each other, the free end of the male coupling member 3 is inserted in the open end of the coupling housing 1, so that the pins 17 will move through the actuating slots and bayonet slots, stopping at the ends of the actuating slots and the "knees" of the bayonet slots. Then, the male coupling member 3 is turned relative to the coupling housing 1, so that the pins 17 are moved into the bottoms of the bayonet slots, thus securing the two coupling members together, while the pins 17 remain stationary in the actuating slots 15, thus turning the valve sleeve 9 to its open position. This proces is reversible for the separation of the coupling members. In order to make it easier to turn the male coupling members 3 in the bayonet socket of the coupling house 1, and at the same time turn the valve sleeve 9 relative to the housing 1, the male coupling member 3 is provided with a rifled or knurled flange 18.

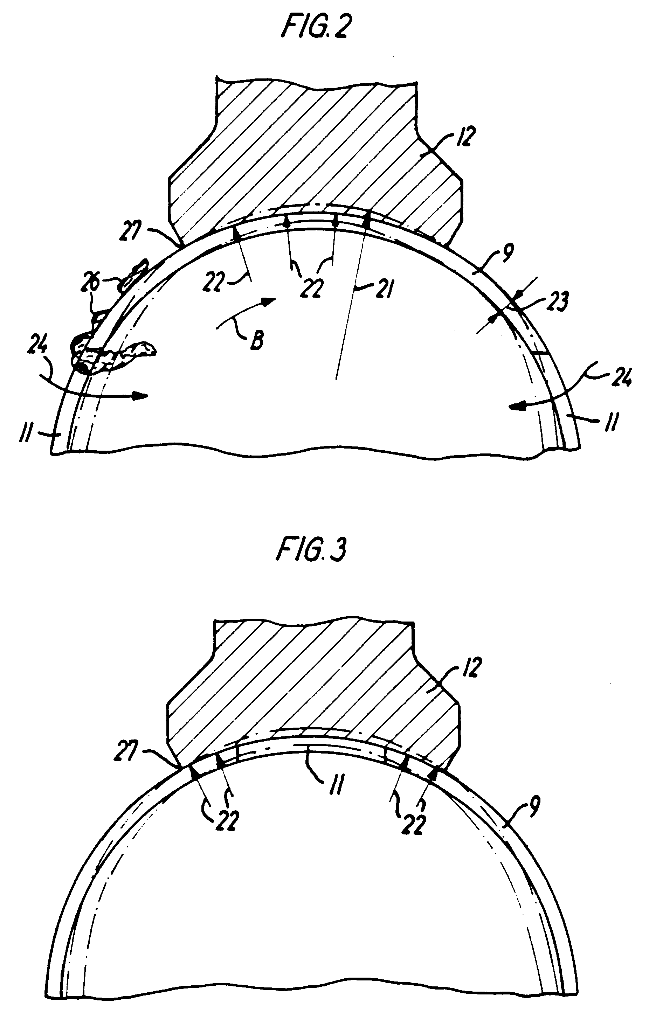

Figure 2 shows a magnified sectional view of the valve sleeve 9 in its open position, in which the valve apertures 11 are open for a urine flow 24. The above-mentioned one end 10 of the valve sleeve 9 with the valve apertures 11 are manufactured with a small wall thickness 23. This makes it possible for this one end 10 of the valve sleeve 9 to be pressed into a slightly oval shape by the bearing and closure members 12, this occuring because the diametral distance between them is less than the outside diameter 21 of the valve sleeve 9 as manufactured. Because of this pressing into oval shape, the valve sleeve 9 exerts a pressure indicated with arrows 22 against the closure elements 12, which pressure partly secures a liquid- tight closing of the valve apertures 11 by the closure elements 12 in the closed position (Figure 3), partly produce the effect, that salt crystals and impurities from the urine, tending to be deposited inter alia at the valve apertures 11 and hence likely to cause valve leakage, are effectively scraped off against the edge 27 of the closure members 12, when the valve sleeve 9 is turned (the arrow B) from the open to the closed position.

## Claims

1. Valve and coupling device for connecting a urine catheter with a urine bag and comprising
a) a first coupling part (1) embracing and being movable relative to a valve member (9) with at least one valve aperture (11) capable of being moved into an open or closed position respectively by movement of the valve member (9) relative to the first coupling member (1) partly embracing it in a liquid-tight manner, and
b) a second coupling member (3),
**characterized** in that the second coupling member (3) is provided with driving and locking members (17) for releasable coupling engagement with the first coupling member (1), said driving and locking members being constituted by the same at least one pin (17) adapted to cooperate with driving slots (15) in the valve member (7) and bayonet slots (16) in the first coupling member (1) in such a manner, that the valve apertures (11) are in a closed position, when the bayonet coupling is in a position for separation, and in an open position, when the bayonet coupling is in a locked-together position.

2. Device according to claim 1, **characterized** in that the valve member (9) abuts with force against a radially projecting element (12) situated in the first coupling member (1), said element (12) having a scraping edge (27) for scraping any impurities (26) off from the valve member (9).

3. Device according to claim 1 or 2, **characterized** in
a) that the valve member (9) is cylindrical and at its periphery cooperates with at least one bearing and closure member (12) in the first coupling part (1), the inscribed circle of said bearing and closure member (12) having a diameter somewhat less than the external diameter (21) of the valve member (9),
b) that the valve member (9) in the region of cooperation is so thin-walled, that it is deformed elastically towards an oval shape between the closure members (12) and hence excerting a sealing pressure (22) against them, said pressure providing a seal, when the valve apertures (11) are in a closed position opposite the closure members (12).

## Patentansprüche

1. Ventil- und Kupplungseinrichtung zur Verbindung eines Harnkatheters mit einem Harnbeutel und umfassend
a) ein erstes Kupplungsteil (1), das ein Ventilorgan (9) mit mindestens einer Ventilöffnung (11) umschliesst und im Verhältnis zu diesem beweglich ist und in eine geöffnete beziehungsweise geschlossene Stellung bewegt werden kann durch Bewegung des Ventilorgans (9) im Verhältnis zu dem es teilweise flüssigkeitsdicht umschliessenden ersten Kupplungsorgan (1), und
b) ein zweites Kupplungsorgan (3),
dadurch **gekennzeichnet,** dass das zweite Kupplungsorgan (3) mit Antriebs- und Schliessorganen (17) zum lösbaren Kupplungseingriff mit dem ersten Kupplungsorgan (1) versehen ist, wobei die genannten Antriebs- und Schliessorgane gebildet werden von mindestens einem Stift (17), der so eingerichtet ist, dass er mit Antriebsschlitzen (15) im Ventilorgan (7) und Bajonettschlitzen (16) in dem ersten Kupplungsorgan (1) auf eine solche Weise zusammenwirkt, dass die Ventilöffnungen (11) sich in geschlossener Stellung befinden, wenn die Bajonettkupplung in Trennungsstellung ist, und in geöffneter Stellung, wenn die Bajonettkupplung in einer Zusammenschlussstellung ist.

2. Einrichtung gemäss Anspruch 1, dadurch **gekennzeichnet,** dass das Ventilorgan (9) an einem in dem ersten Kupplungsorgan (1) befindlichen radial vorspringenden Element (12) stramm anliegt, wobei das genannte Element (12) mit einer Kratzkante (27) versehen ist, um beliebige Verunreinigungen vom dem Ventilorgan (9) abzukratzen.

3. Einrichtung gemäss Anspruch 1 oder 2, dadurch **gekennzeichnet,** dass
a) das Ventilorgan (9) zylindrisch ist und an seinem Umkreis mit zumindest einem Stütz- und Verschlussorgan (12) in dem ersten Kupplungsteil (1) zusammenwirkt, wobei der einbeschriebene Kreis des genannten Stütz- und Verschlussorgans (12) einen Diameter aufweist, der etwas kleiner ist als der Aussendiameter (21) des Ventilorgans (9),
b) dass das Ventilorgan (9) im Bereich des Zusammenwirkens so dünnwandig ist, dass es elastisch auf eine ovale Form zwischen den Vershlussorganen (12) zu verformt wird und folglich einen Dichtungsdruck (22) gegen sie ausübt, wobei genannter Druck eine Dichtung hervorbringt, wenn die Ventilöffnungen (11) sich in einer geschlossenen Stellung gegenüber den Verschlussorganen (12) befinden.

## Revendications

1. Dispositif de soupape et d'accouplement pour relier un cathéter à urine à un sachet à urine et comprenant
a) une première portion d'accouplement (1) embrassant un organe de soupape (9) avec au moins une ouverture de soupape (11) et étant mobile par rapport à celui-ci et qui peut être déplacée vers une position ouverte respectivement fermée par mouvement de l'organe de soupape (9) par rapport au premier organe d'accouplement (1) qui l'embrasse partiellement d'une manière étanche aux liquides, et
b) un deuxième organe d'accouplement (3)
caractérisé en ce que le deuxième organe d'accouplement (3) est muni d'organes d'entraînement et de fermeture (17) pour l'engrènement débrayable avec le premier organe d'accouplement (1), ces organes d'entraînement et de fermeture étant constitués d'au moins une goupille (17) adaptée à coopérer avec des lentes d'entraînement (15) dans l'organe de soupape (7) et des lentes à baionnette (16) dans le premier organe d'accouplement d'une telle manière à ce que les ouvertures de soupape (11) se trouvent dans la position fermée lorsque l'accouplement à baionnette est à position de séparation, et dans la position ouverte lorsque l'accouplement à baionnette est dans une position de fermeture conjointe.

2. Dispositif selon la revendication 1, caractérisé en ce que l'organe de soupape (9) s'appuie solidement sur un élément (12) saillant radialement se trouvant dans le premier organe d'accouplement (1), cet élément (12) ayant un bord grattant (27) pour gratter des impuretés quelconque de l'organe de soupape (9).

3. Dispositif selon l'une des revendications 1 ou 2, caractérisé en ce que
a) l'organe de soupape (9) est cylindrique et qu'il, à sa périphérie, coopère avec au moins un organe de support et de fermeture (12) dans la première portion d'accouplement (1), le cercle inscrit de cet organe de support et de fermeture (12) ayant un diamètre qui est un peu plus moindre que le diamètre extérieur de l'organe de soupape (9),
b) l'organe de soupape (9), dans la région de coopération, a des parois aussi minces qu'il est déformé élastiquement vers une forme ovale entre les organes de fermeture (12) et alors exerce une pression d'étanchement (22) contre elles, cette pression engendrant un étanchement lorsque les ouvertures de soupape (11) se trouvent dans la position fermée opposée aux organes de fermeture (12).
